# EUROPÄISCHE PATENTSCHRIFT

(11) **EP 0 859 804 B1**
(45) Veröffentlichungstag und Bekanntmachung des Hinweises auf die Patenterteilung: **08.03.2000**
(21) Anmeldenummer: 96938122.7
(22) Anmeldetag: 06.11.1996
(51) Int. Cl.: C08G 18/08, C08G 18/12, C08G 18/61, C08L 75/04, A61K 7/06, A61K 7/48, A61K 9/32

(54) **WASSERLÖSLICHE ODER WASSERDISPERGIERBARE POLYURETHANE MIT ENDSTÄNDIGEN SÄUREGRUPPEN, IHRE HERSTELLUNG UND IHRE VERWENDUNG**
WATER-SOLUBLE OR WATER-DISPERSIBLE POLYURETHANES WITH TERMINAL ACID GROUPS, THE PRODUCTION AND THE USE THEREOF
POLYURETHANES HYDROSOLUBLES OU HYDRODISPERSIBLES CONTENANT DES GROUPES ACIDE TERMINAUX, LEUR PRODUCTION ET LEUR UTILISATION

(30) Priorität: 06.11.1995 DE 19541326
(43) Veröffentlichungstag der Anmeldung: 26.08.1998
(73) Patentinhaber: BASF AKTIENGESELLSCHAFT, 67056 Ludwigshafen (DE)
(72) Erfinder: NGUYEN KIM, Son, D-69502 Hemsbach (DE); SPERLING, Karin, D-67433 Neustadt (DE)
(74) Vertreter: Riedl, Peter, Dr.
(86) Internationale Anmeldenummer: EP9604858
(87) Internationale Veröffentlichungsnummer: WO9717386

(56) Entgegenhaltungen:
- EP-A- 0 636 361
- EP-A- 0 647 667
- WO-A-95/08583
- DE-A- 1 954 090

## Beschreibung

Die vorliegende Erfindung betrifft wasserlösliche oder wasserdispergierbare Polyurethane mit endständigen Säuregruppen, ihre Herstellung und ihre Verwendung in der Kosmetik.

In Wasser lösliche oder in Wasser dispergierbare Polymere, beispielsweise Polyester, Polyamide oder Polyurethane, gewinnen aufgrund ihrer besonders niedrigen Viskosität in Wasser/Ethanol Produkteigenschaften immer mehr an Bedeutung. So sind wasserlösliche Polyurethane, die Carboxylgruppen aufweisende Diole einpolymerisiert enthalten, aus der US-A-3,412,054 und 3,658,939 bekannt. Sie werden als Klebstoff, Beschichtungsmittel und in Drucktinten verwendet. Sulfonat- und/oder Carboxylatgruppen enthaltende ?olyurethane, die in Wasser dispergierbar sind, sind aus der DE-A-15 70 615 bekannt. Sie werden beispielsweise zur Beschichtung und zum Imprägnieren von Textilien, Leder, Papier, Holz und Metallen verwendet. Aus den Schutzrechten US-A-4,300,580, US-A-3,734,874, DE-A-26 33 418 und WO-A-89/07118 sind NaSO₃-Gruppen enthaltende Polyester bekannt, deren Hauptkette durch Kondensationsreaktion aufgebaut ist und die durch Hydrolyse der Estergruppierungen zu kürzeren Segmenten abgebaut werden können.

Weiter ist bekannt, daß Maleinsäureanhydrid und Trimellitsäureanhydrid zur Herstellung von wasserlöslichen Estern verwendet werden können. Die Anhydridgruppierung stellt Carboxylgruppen zur Verfügung, welche durch Neutralisation mit Aminen, Metallhydroxiden und Metallcarbonaten in Carboxylatgruppen überführt werden, wodurch Wasserlöslichkeit erzielt wird. Aus der DE-A-26 37 167 und der US-A-3,523,998 ist bekannt, daß auch Polycarbonsäuren und ihre Anhydride als Polymerisatkomponenten dazu beitragen können, Polyester wasserlöslich zu machen. Die DE-A-21 44 878 beschreibt Polyurethane, bei denen es sich um Umsetzungsprodukte aus aufgeschlossenem Kasein, in Wasser dispergierbaren Polyurethanen und Formaldehyd handelt. Als Polyurethankomponente wird unter anderem ein Latex eingesetzt, der erhältlich ist durch Umsetzung eines Polyurethanprepolymers mit einer Natriumtaurinlösung. Der Latex besitzt ein relativ niedriges Molekulargewicht und einen geringen Gehalt an ionogenen bzw. ionischen Gruppen, weil er neben den Sulfonatgruppen aus dem Taurin keine weiteren ionogenen bzw. ionischen Gruppen enthält. Ein aus dem Latex erhaltener Film ist daher in Wasser ohne Dispergiermittel nicht löslich. Der erhaltene Latex wird dann mit Kasein und Formaldehyd zu dem erwähnten Umsetzungsprodukt zur Reaktion gebracht. Eine kosmetische Anwendung derartiger Polymere ist bisher jedoch noch nicht beschrieben.

In der Kosmetik werden Filmbildnerpolymere zur Festigung, Strukturverbesserung und Formgebung der Haare verwendet. Die Haarbehandlungsmittel enthalten im allgemeinen eine Lösung des Filmbildners in einem Alkohol oder einem Gemisch aus Alkohol und Wasser.

Die US-A-4,743,673 beschreibt hydrophile Polyurethanpolymere mit Carboxygruppen im Polymerrückgrat. Diese Polyurethane sind aufgebaut aus einer Polyolkomponente,bei der es sich um ein Alkylenglycol, ein Polyoxyalkylenglycol, oder ein lineares Polyesterdiol handeln kann, einer Carbonsäureesterkomponente mit Hydroxy- oder Aminogruppen und einem organischen Isocyanat oder Isocyanat-Vorläufer. Das Polyurethan enthält also an das Polymerrückgrat gebundene Estergruppen, die anschließend durch 30- bis 60-minütiges Erhitzen mit einer starken Base, wie Natrium- oder Kaliumhydroxid, unter Rückfluß verseift werden. Das erhaltene Produkt ist sowohl in Wasser als auch in Ethanol nicht mehr klar löslich. Insbesondere bei Verwendung eines Polyesterdiols als Polyolkomponente erfolgt aufgrund der Behandlung mit der starken Base unter Rückflußbedingungen nicht nur eine Verseifung der Estergruppen der Carbonsäureesterkomponente, sondern auch eine Verseifung der in der Polyurethankette enthaltenen Estergruppierungen. Es kommt somit zur Spaltung der Polyurethankette und zu einer drastischen Verringerung des Molekulargewichts der Polyurethane. Eine Anwendung der Polyurethane in Haarsprays ist zwar erwähnt. In der Praxis sind die mit diesen Polyurethanen erhaltenen Filme für die Haarkosmetik aber nicht brauchbar, weil sie entweder wasserunlöslich sind oder ein zu geringes Molekulargewicht und somit unzureichende Festigungswirkung besitzen.

Die DE-A-42 25 045 beschreibt die Verwendung von wasserlöslichen oder in Wasser dispergierbaren, anionischen Polyurethanen als Haarfestiger. Diese Polyurethane sind aufgebaut aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat.

Sie besitzen eine Glastemperatur von mindestens 15°C und Säurezahlen von 12 bis 150. Als Komponente a) kommen vorzugsweise Polyethylenglycol, Neopentylglycol und Polyesterole zur Anwendung. Bevorzugte Komponenten (b) sind Dimethylolpropansäure, ein Kondensat aus Pyromellithsäuredianhydrid und Neopentylglycol und ein Kondensat aus 5-Natriumsulfonatoisophthalsäure mit Neopentylglycol.

Die DE-A-42 41 118 beschreibt die Verwendung von kationischen Polyurethanen und Polyharnstcffen als Hilfsmittel in kosmetischen und pharmazeutischen Zubereitungen. Sie finden insbesondere Anwendung als Filmbildner in Haarfestigern. Sie sind aufgebaut aus
a) mindestens einem Diisocyanat, welches bereits vorher mit einer oder mehreren Verbindungen, die zwei oder mehrere aktive Wasserstoffatome pro Molekül enthalten, umgesetzt sein kann und
b) mindestens einem Diol, primären oder sekundären Aminoalkohol, primären oder sekundären Diamin oder primären oder sekundären Triamin mit einem oder mehreren tertiären, quartären oder protonierten tertiären Aminstickstoffatomen.

Die Polymere besitzen eine Glasübergangstemperatur von mindestens 25°C und eine Aminzahl von 50 bis 200, bezogen auf die nicht-quaternisierten oder protonierten Verbindungen.

Die EP-A-619 111 beschreibt die Verwendung von Polyurethanen mit Carboxylatgruppen in Haarfixierungsmitteln. Als Verbindung, welche die Carboxylatgruppen zur Verfügung stellt, enthalten diese Polyurethane eine Verbindung der Formel 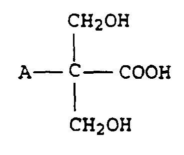 worin A für ein Wasserstoffatom oder eine C₁-C₂₀-Alkylgruppe steht. Zumindest ein Teil der Carbonsäuregruppen wird dabei mit einer organischen oder anorganischen Base neutralisiert, um die Anzahl an Carboxylatgruppen zur Verfügung zu stellen, die erforderlich ist, um das Polyurethan in Wasser oder einem Gemisch aus Wasser und einem polaren organischen Lösungsmittel löslich zu machen.

Haarfestigungsmittel werden im allgemeinen in Form von wäßrig-alkoholischen Lösungen auf die Haare aufgesprüht. Nach dem Verdampfen des Lösungsmitttels werden die Haare an den gegenseitigen Berührungspunkten vom zurückbleibenden Polymer in der gewünschten Form gehalten. Die Polymere sollen einerseits so hydrophil sein, daß sie aus dem Haar ausgewaschen werden können, andererseits aber sollen sie hydrophob sein, damit die mit den polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht (K-Wert >25) und eine relativ hohe Glastemperatur (mindestens 15°C) besitzen. Polyurethane, welche diese Forderungen erfüllen, sind aber aufgrund des höheren Molekulargewichtes schlechter auswaschbar.

Bei der Formulierung von Haarfestigern ist außerdem zu berücksichtigen, daß aufgrund der Umweltbestimmungen zur Kontrolle der Emission flüchtiger organischer Verbindungen (VOC = volatile organic compounds) in die Atmosphäre eine Verringerung des Alkohol- und Treibmittelgehalts erforderlich ist.

Die in den oben erwähnten Publikationen beschriebenen Polymere erfüllen diese einander entgegenstehenden Anforderungen nur teilweise. So besitzen die in der DE-A-42 25 045 und 42 41 118 sowie in der EP-A-619 111 beschriebenen Polymere aufgrund ihres hohen Molekulargewichtes einerseits die gewünschte Festigerwirkung. Andererseits aber sind sie nur unzureichend auswaschbar. Die in der US 4,743,673 beschriebenen Polymere wiederum besitzen aufgrund ihres durch Verseifen der Estergruppen bewirkten geringen Molekulargewichtes nicht die erforderliche Festigerwirkung.

Die EP-A-636 361 beschreibt kosmetische Mittel, welche als Filmbildner ein Polykondensat enthält, das mindestens eine Polysiloxaneinheit und mindestens eine Polyurethan- und/oder Polyharnstoffeinheit mit anionischen oder kationischen Gruppen umfaßt. Auch die Auswaschbarkeit dieser Filmbildner ist nicht zufriedenstellend.

Der vorliegenden Erfindung liegt daher die Aufgabe zugrunde, Haarbehandlungsmittel zur Verfügung zu stellen, die einerseits als Haarfestiger brauchbar sind, andererseits aber auch verbesserte Auswaschbarkeit (Redispergierbarkeit) besitzen.

Überraschenderweise wurde nun gefunden, daß diese Aufgabe durch wasserlösliche bzw. durch Wasser dispergierbare Polyurethane gelöst wird, welche das Umsetzungsprodukt aus einem Urethanpräpolymer mit endständigen Isocyanatgruppen mit einem primären oder sekundären Amin, das mindestens eine ionogene bzw. ionische Gruppe aufweist, darstellen.

Gegenstand der vorliegenden Erfindung sind daher wasserlösliche oder wasserdispergierbare Polyurethane aus
A) einem in Wasser löslichen oder dispergierbaren Polyurethanpräpolymer mit endständigen Isocyanatgruppen und
B) mindestens einem primären oder sekundären Amin, das mindestens eine ionogene bzw. ionische Gruppe aufweist,
sowie die Salze davon.

Das primäre oder sekundäre Amin reagiert mit den endständigen Isocyanatgruppen des Urethanpräpolymers, so daß das Amin über eine Harnstoffgruppierung an das Polyurethan gebunden ist. Die erfindungsgemäßen Polyurethane weisen also endständige, von dem Amin abgeleitete Gruppen mit jeweils mindestens einer ionogenen bzw. ionischen Gruppe auf. Sie besitzen vorzugsweise einen K-Wert von 15 bis 100, insbesondere 20 bis 50 und vorzugsweise eine Glasübergangstemperatur T_{g} von 15 bis 150, insbesondere 25 bis 100.

Wenn die Polyurethane Carbonsäure- oder Sulfonsäuregruppen enthalten, liegt die Säurezahl vorzugsweise im Bereich von 12 bis 150, insbesondere 30 bis 90.

Wenn die Polyurethane Amingruppen bzw. protonierte oder quaternisierte Amingruppen enthalten, liegt die Aminzahl vorzugsweise im Bereich von 30 bis 180, insbesondere 50 bis 120.

Erfindungsgemäß brauchbare Polyurethan-Präpolymere sind bekannt. Es handelt sich dabei um Polyurethane, welche ionogene bzw. ionische, an die Polymerkette gebundene Gruppen aufweisen, damit die Polyurethane in Wasser löslich bzw. dispergierbar sind. Bei diesen Gruppen handelt es sich um Carbonsäuregruppen und/oder stickstoffhaltige Gruppen (Amine) bzw. Carboxylatgruppen und/oder quaternisierte oder protonierte Gruppen. Derartige Polyurethan-Präpolymere werden gebildet aus:
a) mindestens einer Verbindung, die zwei oder mehrere aktive Wasserstoffatome pro Molekül enthält,
b) mindestens einer Verbindung, die zwei oder mehrere aktive Wasserstoffatome und mindestens eine ionogene bzw. ionische Gruppe pro Molekül aufweist, und
c) mindestens einem Diisocyanat.

Bei der Komponente (a) handelt es sich um Diole, Diamine, Aminoalkohole, Polyetherdiole und Polyesterdiole mit einem zahlenmittleren Molekulargewicht von jeweils bis zu 3000 oder deren Mischungen, wobei bis zu 3 Mol-% der genannten Verbindungen durch Triole oder Triamine ersetzt sein können. Besonders bevorzugt kommt als Komponente (a) ein Diolgemisch zur Anwendung, das mindestens 30 Gew.-% und insbesondere 40 bis 80 Gew.-%, bezogen auf das Gesamtgewicht der Komponenten (a) und (b), eines Polyesterdiols umfaßt.

Brauchbare Diole sind z.B. Ethylenglycol, Propylenglycol, Butylenglycol, Neopentylglycol, Polyetherole, wie Polyethylenglycole mit Molekulargewichten bis zu 3000, Blockcopolymerisate aus Ethylenoxid und Propylenoxid mit zahlenmittleren Molekulargewichten von bis zu 3000 oder Blockcopolymerisate aus Ethylenoxid, Propylenoxid und Butylenoxid, die die Alkylenoxideinheiten statistisch verteilt oder in Form von Blöcken einpolymerisiert enthalten. Bevorzugt sind Ethylenglycol, Neopentylglycol, Di-, Tri-, Tetra-, Penta oder Hexaethylenglycol.

Geeignete Aminoalkohole sind z.B. 2-Aminoethanol, 2-(N-Methylamino)ethanol, 3-Aminopropanol oder 4-Aminobutanol.

Geeignete Diamine sind z.B. Ethylendiamin, Propylendiamin, 1,4-Diaminobutan und 1,6-Diaminohexan sowie α,ω-Diamine, die durch Aminierung von Polyalkylenoxiden mit Ammoniak herstellbar sind.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, sowie Poly(meth)acrylatdiole der Formel 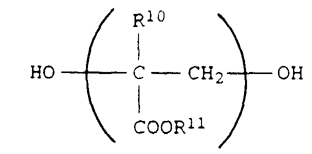 worin R¹⁰ für H oder CH₃ steht und R¹¹ für C₁-C₁₈-Alkyl (insbesondere C₁-C₁₂- oder C₁-C₈-Alkyl) steht, die eine Molmasse von bis zu etwa 3000 aufweisen. Derartige Diole sind auf übliche Weise herstellbar und im Handel erhältlich (Tegomer®-Typen MD, BD und OD der Fa. Goldschmidt).

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 50 bis 90 Mol-% und bevorzugt 70 bis 85 Mol-%, des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/1,6-Hexandiol, 5-NaSO₃-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/Dimethylolcyclohexan.

Die Polyesterdiole haben vorzugsweise eine Molmasse im Bereich von etwa 400 bis 5000, insbesondere 500 bis 3000.

Polyurethane auf Basis dieser Polyesterdiole und aliphatischer Diisocyanate sind von besonderem Vorteil, weil sie biologisch abbaubar sind.

Als Komponente (a) brauchbar sind auch Silikonverbindungen der Formel 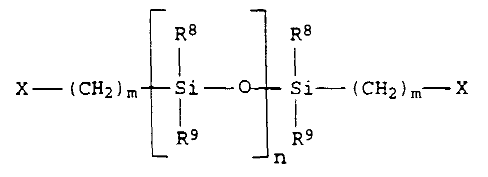
worin R⁸ und R⁹, die gleich oder verschieden sein können, für C₁-C₄-Alkyl, Benzyl oder Phenyl, vorzugsweise Methyl, stehen,
die Reste X, die gleich oder verschieden sein können, für OH oder NH₂ stehen,
m für 2 bis 8 steht, und
n für 3 bis 50, insbesondere 3 bis 30, steht.

Diese Silikonverbindungen können in einer Menge von bis zu 50 Gew.-%, insbesondere bis zu 30 Gew.-%, bezogen auf das Gesamtgewicht der Komponenten (a) und (b) zur Anwendung kommen.

Die polysiloxanhaltigen Polyurethane wirken als Lösungsvermittler für hydrophobe Produkte, insbesondere Silikone und ermöglichen daher deren Aufnahme in die Haarbehandlungsmittel. Die Silikone machen die Haare glänzend, geschmeidig und glatt und sind vorzugsweise in Mengen bis zu 0,2 Gew.-%, bezogen auf das Gesamtgewicht des Mittels, enthalten. Vorzugsweise verwendet man nichtflüchtige Silikone, insbesondere solche auf Poly(dimethylsiloxan)-Basis. Geeignete Silikone sind insbesondere Dimethicone, z.B. die Abil®-Typen der Fa. Goldschmidt.

Wenn als Komponente (b) Verbindungen mit Carboxylatgruppen oder Sulfonatgruppen eingesetzt werden, erhält man anionische Polyurethane. Als Komponente (b) ist Dimethylolpropansäure besonders bevorzugt. Brauchbar sind auch Verbindungen der Formeln 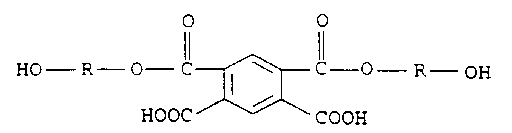 und/oder 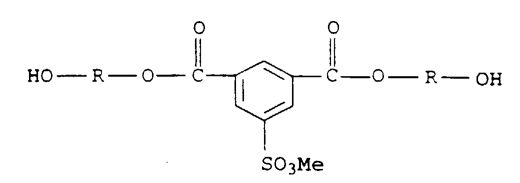 worin R jeweils für eine C₂-C₁₈-Alkylengruppe steht und Me für Na oder K steht.

Als Komponente (b) brauchbar sind auch Verbindungen der Formel

H₂N(CH₂)ₙ-NH-(CH₂)ₘ-COO⁻M⁺

H₂N(CH₂)ₙ-NH-(CH₂)ₘ-SO₃⁻ M⁺

worin m und n unabhängig voneinander für eine ganze Zahl von 1 bis 8, insbesondere 1 bis 6, stehen und M für Li, Na oder K steht.

Wenn man als Komponente (b) Verbindungen mit stickstoffhaltigen Gruppen verwendet, erhält man kationische Polyurethane. Brauchbare Komponenten (b) sind z.B. Verbindungen der allgemeinen Formeln 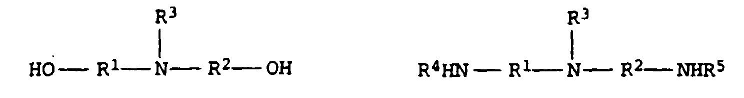 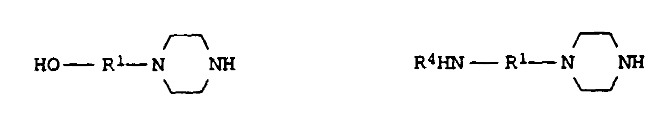 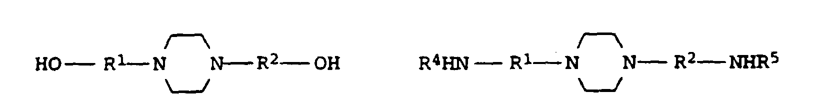 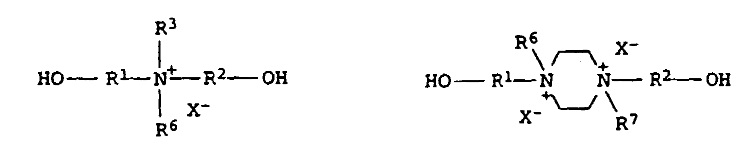 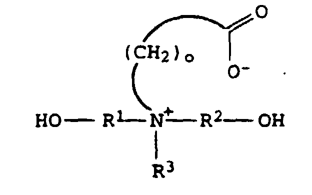 worin
R¹ und R², die gleich oder verschieden sein können, für C₂-C₈-alkylen stehen,
R³, R⁶ und R⁷, die gleich oder verschieden sein können, für C₁-C₆-Alkyl, Phenyl oder Phenyl-C₁-C₄-alkyl stehen,
R⁴ und R⁵, die gleich oder verschieden sein können, für H oder C₁-C₆-Alkyl stehen,
o für 1, 2 oder 3 steht,
X^{⊖} für Chlorid, Bromid, Jodid, C₁-C₆-Alkylsulfat oder SO₄²⁻/2 steht.

Bei der Komponente (c) handelt es sich um Diisocyanate, ausgewählt aus Isophorondiisocyanat, Methylendiphenyldiisocyanat (MDI) und/oder Toluylendiisocyanat.

Weitere erfindungsgemäße Polyurethanprepolymere sind beispielsweise beschrieben in der US-A-3,475,206 und 3,412,054 sowie in der DE-A-15 70 615. Vorzugsweise verwendet man jedoch die in der DE-A-42 25 045, DE-A-42 41 118 und EP-A-619 111 beschriebenen Polyurethane. Es handelt sich dabei um die folgenden:
1. In Wasser lösliche oder dispergierbare anionische Polyurethane aus
   a) mindestens einer Verbindung, die zwei oder mehrere aktive Wasserstoffatome pro Molekül enthält,
   b) mindestens einem Säure- oder Salzgruppen enthaltenen Diol und
   c) mindestens einem Diisocyanat,
      die eine Glasübergangstemperatur von mindestens 15°C und eine Säurezahl im Bereich von 12 bis 150 besitzen, und die Salze davon.

   Die Komponenten (a), (b) und (c) sind wie oben beschrieben.
   Diese Polymere und ihre Herstellung sind in der DE-A-42 25 045 näher beschrieben, auf die hiermit in vollem Umfang Bezug genommen wird.
2. In Wasser lösliche dispergierbare kationische Polyurethane und Polyharnstoffe, wobei man die oben angegegebenen Komponenten (a) bis (c) verwendet. Als Komponente (c) kann auch ein Diisocyanat verwendet werden, welches bereits vorher mit einer oder mehreren Verbindungen, die 2 oder mehrere aktive Wasserstoffatome pro Molekül enthalten, d.h. mit Komponenten (a), umgesetzt worden sein kann.
   Derartige Polyurethane und ihre Herstellung sind in der DE-A-42 41 118 näher beschrieben, auf die hiermit in vollem Umfang Bezug genommen wird.
3. Lineare Polyurethane mit Carboxylatgruppen aus
   a) 10 bis 90 Gew.-%, bezogen auf das Gewicht des Polyurethans, einer oder mehrerer organischer Verbindungen mit nicht mehr als zwei aktiven Wasserstoffatomen,
   b) einer 2,2-Hydroxymethyl-substituierten Carbonsäure der Formel 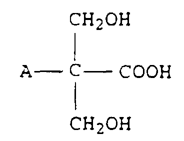 worin A für ein Wasserstoffatom oder eine C₁-C₂₀-Alkylgruppe steht, die in einer Menge verwendet wird, welche ausreicht, daß in dem Polyurethan 0,35 bis 2,25 milliäquivalente Carboxylgruppen pro g Polyurethan vorhanden sind,
   c) einem oder mehreren organischen Diisocyanaten.
      Die Komponenten a) und c) sind wie oben angegeben.
      Die im Polyurethan enthaltenen Carboxylgruppen werden abschließend mit einer geeigneten Base zumindest teilweise neutralisiert. Diese Polymere und ihre Herstellung sind in der EP-A-619 111 näher beschrieben, auf die hiermit in vollem Umfang Bezug genommen wird.

Die Polyurethan-Präpolymere sind dadurch erhältlich, daß man die Verbindungen der Gruppen a) und b) unter einer Inertgasatmosphäre in einem inerten Lösemittel oder ohne Lösungsmittel (in der Schmelze) bei Temperaturen von 70 bis 130°C mit den Verbindungen der Gruppe c) umsetzt. Dabei werden die Komponenten in solchen Mengen eingesetzt, daß das Verhältnis von NCO-Äquivalent zu OH-Äquivalent größer als 1 ist und bis zu 1,2 betragen kann. Vorzugsweise liegt das Verhältnis im Bereich von 1,02 bis 1,12. Die Säurezahl der Polyurethane wird von der Zusammensetzung und der Konzentration der Verbindungen der Komponente (b) in der Mischung aus den Komponenten (a)+(b) bestimmt. Die Polyurethane haben K-Werte nach H. Fikentscher (bestimmt in 0,1 gew.-%igen Lösungen in N-Methylpyrrolidon bei 25°C und pH 7) von 15 bis 100, vorzugsweise 25 bis 50.

Gemäß einer bevorzugten Ausführungsform handelt es sich bei der Komponente (B) um ein Amin der Formel:

R¹⁰HN - Y - SO₃H

worin
Y für o-, m- oder p-Phenylen oder geradkettiges oder verzweigtes C₂-C₆-Alkylen steht, das gegebenenfalls durch 1, 2 oder 3 Hydroxygruppen substituiert ist, und
R¹⁰ für ein Wasserstoffatom, eine C₁-C₁₂-Alkylgruppe (vorzugsweise C₁-C₁₀- und insbesondere C₁-C₆-Alkylgruppe) oder eine C₅-C₆-Cycloalkylgruppe steht, wobei die Alkylgruppe oder die Cycloalkylgruppe gegebenenfalls durch 1, 2 oder 3 Hydroxygruppen, Carboxylgruppen oder Sulfonsäuregruppen substituiert sein kann.

Besonders bevorzugt handelt es sich bei dem Amin der obigen Formel um Taurin, N-(1,1-Dimethyl-2-hydroxyethyl)-3-amino-2-hydroxypropansulfonsäure oder 4-Aminobenzolsulfonsäure.

Gemäß einer weiteren bevorzugten Ausführungsform handelt es sich bei dem Amin um eine übliche α-, β- oder γ-Aminosäure, beispielsweise Glycin, Alanin, Valin, Leucin, Isoleucin, Phenylalanin, Thyrosin, Prolin, Hydroxyprolin, Serin, Threonin, Methionin, Cystein, Tryptophan oder β-Alanin. Bevorzugte Aminsäuren sind Asparaginsäure und Glutaminsäure.

Die Herstellung der erfindungsgemäßen Polyurethane erfolgt durch Umsetzung des Polyurethanpräpolymers mit dem primären oder sekundären Amin mit einer ionogenen bzw. ionischen Gruppe, in einem geeigneten inerten Lösungsmittel oder ohne Lösungsmittel (in der Schmelze). Man verwendet das Amin in einer Menge, daß die freien Isocyanatgruppen des Polyurethan-Prepolymer zumindest teilweise, vorzugsweise jedoch vollständig umgesetzt werden. Die Umsetzung erfolgt in einer Weise, wie sie aus dem Stand der Technik für das Abstoppen der Polyurethanpolymerisation mit Aminen bekannt ist. Gegebenenfalls noch vorhandene Isocyanatgruppen werden abschließend durch Zusatz von Aminen, z.B. 2-Amino-2-methyl-1-propanol, inaktiviert.

Nach Ersatz des Lösungsmittels durch Wasser erhält man eine Lösung oder Dispersion des Polymers, aus der, falls gewünscht, das Polymer in üblicher Weise gewonnen werden kann, z.B. durch Sprühtrocknung.

Vorzugsweise setzt man das Amin in Form einer wäßrigen oder wäßrig-alkoholischen Lösung mit einem pH >7,5 ein, um die Reaktionsfähigkeit des Amins zu erhöhen. Die Einstellung des pH's kann in üblicher Weise erfolgen, beispielsweise mit einem Alkalihydroxid, wie NaOH oder KOH oder vorzugsweise mit einem tertiären Amin, wie Triethylamin, einem C₁-C₆-Alkyldiethanolamin, z.B. Methyl- oder Ethyldiethanolamin oder einem Di-C₁-C₆-Alkylethanolamin.

Die Säuregruppen enthaltenden Polyurethane sind nach Neutralisation (teilweise oder vollständig) wasserlöslich bzw. ohne Zuhilfenahme von Emulgatoren in Wasser dispergierbar. In aller Regel weisen die erhaltenen Salze der Polyurethane eine bessere Wasserlöslichkeit oder Dispergierbarkeit in Wasser auf als die nicht neutralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Besonders für den Einsatz in Haarbehandlungsmitteln haben sich zur Neutralisation der Säuregruppen enthaltenden Polyurethane 2-Amino-2-Methylpropanol, Diethylaminopropylamin und Triisopropanolamin bewährt. Die Neutralisation der Säuregruppen enthaltenden Polyurethane kann auch mit Hilfe von Mischungen mehrerer Basen vorgenommen werden, z.B. Mischungen aus Natronlauge und Triisopropanolamin. Die Neutralisation kann je nach Anwendungszweck partiell z.B. zu 20 bis 40 % oder vollständig, d.h. zu 100 % erfolgen.

Sind die erfindungsgemäßen Verbindungen wasserdispergierbar, können sie in Form von wäßrigen Mikrodispersionen mit Teilchendurchmessern von üblicherweise 5 bis 100 nm, insbesondere 10 bis 80 nm, und Feststoffgehalten von üblicherweise 1 bis 40 Gew.-%, insbesondere 3 bis 30 Gew.-%, zur Anwendung gebracht werden. Diese Mikrodispersionen benötigen in der Regel keine Emulgatoren oder Tenside zu ihrer Stabilisierung.

Die Amingruppen bzw. protonierte oder quaternisierte Amingruppen enthaltenden Polyurethane und Polyharnstoffe sind aufgrund ihrer kationischen Gruppierungen in der Regel leicht alkohol- und wasserlöslich oder zumindest ohne Zuhilfenahme von Emulgatoren in Alkohol und Wasser dispergierbar. Geladene kationische Gruppierungen lassen sich aus den vorliegenden tertiären Aminstickstoffatomen entweder durch Protonierung, z.B. mit Carbonsäuren wie Milchsäure, oder durch Quaternisierung, z.B. mit Alkylierungsmitteln wie C₁- bis C₄-Alkylhalogeniden oder -sulfaten in den Polyharnstoffen erzeugen. Beispiele solcher Alkylierungsmittel sind Ethylchlorid, Ethylbromid, Methylchlorid, Methylbromid, Dimethylsulfat und Diethylsulfat.

Die erfindungsgemäßen Polyurethane sind als Hilfsmittel in der Kosmetik und Pharmazie, insbesondere als Beschichtungsmittel für keratinhaltige Oberflächen (Haar, Haut und Nägel) und als Überzugsmittel und/oder Bindemittel für feste Arzneiformen brauchbar. Außerdem sind sie als Beschichtungsmittel für die Textil-, Papier-, Druck- und Klebstoff-Industrie brauchbar. Sie sind insbesondere in der Haarkosmetik brauchbar. Zur Anwendung als Haarfestiger sind Polyurethane bevorzugt, die mindestens 30 Gew.-% Polyesterdiolkomponente umfassen und deren Glasübergangstemperatur Tg ≥ 25°C ist. Daneben können die Polymere auch in Cremes und als Tablettenüberzugsmittel und Tablettenbindemittel verwendet werden.

Gegenstand der vorliegenden Erfindung ist auch ein kosmetisches oder pharmazeutisches Mittel, das die erfindungsgemäßen Polyurethane enthält. Im allgemeinen enthält das Mittel die Polyurethane in einer Menge im Bereich von 0,2 bis 20 Gew.-%, bezogen auf das Gesamtgewicht des Mittels.

Vorzugsweise handelt es sich um Haarbehandlungsmittel. Diese liegen üblicherweise in Form einer wäßrigen Dispersion oder in Form einer alkoholischen oder wäßrig-alkoholischen Lösung vor. Beispiele geeigneter Alkohole sind Ethanol, Propanol, Isopropanol etc.

Weiter enthalten die erfindungsgemäßen Haarbehandlungsmittel im allgemeinen übliche kosmetische Hilfsstoffe, beispielsweise Weichmacher, wie Glycerin und Glycol; Emollienzien; Parfüms; UV-Absorber; Farbstoffe; Verdickungsmittel; antistatische Mittel; Mittel zur Verbesserung der Kämmbarkeit; Konservierungsmittel; und Schaumstabilisatoren.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel wird so gering wie möglich gehalten, um den VOC-Gehalt nicht unnötig zu erhöhen. Sie beträgt im allgemeinen nicht mehr als 40 Gew.-%, bezogen auf das Gesamtgewicht des Mittels.

Die erfindungsgemäßen Polyurethane können auch in Kombination mit anderen Haarpolymeren in den Mitteln zur Anwendung kommen. Solche Polymere sind insbesondere:
- nicht-ionische, wasserlösliche bzw. wasserdispergierbare Polymere oder Oligomere, wie Polyvinylcaprolactam, z.B. Luviskol Plus (BASF), oder Polyvinylpyrrolidon und deren Copolymere, insbesondere mit Vinylestern, wie Vinylacetat, z.B. Luviskol VA 37 (BASF); Polyamide, z.B. auf Basis von Itaconsäure und aliphatischen Diaminen
- amphotere oder zwitterionische Polymere, wie die unter den Bezeichnungen Amphomer® (Delft National) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind.
- wasserlösliche oder wasserdispergierbare Polymere gleicher Ionogenität wie die erfindungsgemäßen Polymere, d.h.
   anionische erfindungsgemäße Polyurethane und anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer, Acrylsäure/Ethylacrylat/N-tert.Butylacrylamid-Terpolymere, die beispielsweise unter der Bezeichnung Ultrahold® strong (BASF) vertrieben werden sowie Luvimer® (BASF, Terpolymer aus t-Butylacrylat, Ethylacrylat und Methacrylsäure) oder
   kationische erfindungsgemäße Polyurethane und kationische (quaternisierte) Polymere, z.B. Luviquat® (Copolymer aus Vinylpyrrolidon und Vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.
- neutrale, siloxanhaltige, wasserlösliche oder -dispergierbare Polymere, z.B. Polyethersiloxane, wie Tegopren® (Fa. Goldschmidt).

Die anderen Haarpolymere sind vorzugsweise in Mengen bis zu 10 Gew.-%, bezogen auf das Gesamtgewicht des Mittels enthalten. Ein besonders bevorzugtes Haarbehandlungsmittel enthält:
a) mindestens ein siloxanfreies, in Wasser lösliches oder dispergierbares Haarpolymer, insbesondere Haarfestigerpolymer, wobei es sich sowohl um ein erfindungsgemäßes Polyurethan als auch um ein anderes Haarpolymer handeln kann,
b) mindestens ein wie oben definiertes polysiloxanhaltiges Polyurethan und
c) ein wasserunlösliches, nicht-flüchtiges Silicon, insbesondere ein Polydimethylsiloxan, z.B. die Abil-Typen der Fa. Goldschmidt.

Das Mittel enthält vorzugsweise 0,1-10 Gew.-% der Komponente a), 0,1-15 Gew.-% der Komponente b) und 0,0001 bis 0,2 Gew.-% der Komponente c).

Die erfindungsgemäßen Polyurethane und Mittel besitzen den Vorteil, daß sie einerseits den Haaren die gewünschte Festigkeit verleihen und andererseits die Polymere leichter auswaschbar (redispergierbar) sind als die Polymere des Standes der Technik. Darüber hinaus lassen sich Haarbehandlungsmittel mit einem VOC-Gehalt von weniger als 60 Gew.-% und auch rein wäßrige Formulierungen herstellen, selbst wenn sie als Haarspray formuliert sind.

Die nachfolgenden Beispiele erläutern die Erfindung.

### Beispiel 1

### Polyurethanherstellung:

In einem 4-Halskolben, der mit Rührer, Tropftrichter, Thermometer, Rückflußkühler und einer Vorrichtung für das Arbeiten unter Stickstoff ausgestattet war, wurden 0,5 Mol Polyesterdiol (M_{w} = 1000 g/Mol), (hergestellt aus Isophthalsäure, Adipinsäure und Hexandiol, 0,6 Mol Diethylengiycol und 1,25 Mol Dimethylolpropansäure in Methylethylketon (ca. 50%ige Lösung) unter Erhitzen auf eine Temperatur von 80°C und unter Rühren gelöst. Sobald sich alles gelöst hatte, wurde das Reaktionsgemisch auf ca. 50°C abgekühlt. Anschließend wurden unter Rühren 2,5 Mol Isophorondiisocyanat zugetropft, wobei die Reaktionstemperatur anstieg. Bei einer Innentemperatur von 90°C wurde das Reaktionsgemisch dann so lange gerührt, bis der Isocyanatgruppengehalt des Gemisches praktisch konstant blieb. Danach kühlte man das Reaktionsgemisch auf Umgebungstemperatur ab und tropfte bei dieser Temperatur 0,3 Mol (a) Asparaginsäure oder (b) Glutaminsäure oder (c) Taurin jeweils in Form einer 50%igen wäßrigen Aminosäure-Diethylethanolamin-Lösung, wobei das Diethylethanolamin in zur Aminosäure äquimolarer Menge zur Anwendung kam. Das Reaktionsgemisch wurde dann noch so lange bei Umgebungstemperatur gerührt, bis der Isocyanatgruppengehalt 0 war. Anschließend gab man Wasser zum Reaktionsgemisch und neutralisierte das Reaktionsprodukt mit 2-Amino-2-methylpropanol. Das Methylethylketon wurde dann im Vakuum bei 40°C abdestilliert, wobei man eine wäßrige Dispersion des Polyurethans erhielt, welche für die in den nachfolgenden Beispielen 3 und 4 beschriebenen Versuche verwendet wurde.

### Beispiel 2 (Vergleichsbeispiel)

### Polyurethanherstellung (ohne Zugabe einer Aminosäure)

In einem Vierhalskolben, der mit Rührer, Tropftrichter, Thermometer, Rückflußkühler und einer Vorrichtung für das Arbeiten unter Stickstoff ausgestattet war, wurden 0,5 Mol Polyesterdiol (M_{w} = 1000 g/mol), hergestellt aus Isophthalsäure, Adipinsäure und Hexandiol, 0,5 Mol Diethylenglykol und 1,25 Mol Dimethylolpropansäure in Methylethylketon (ca. 50 %ige Lösung) unter Erhitzen auf eine Temperatur von 80°C und unter Rühren gelöst. Sobald sich alles gelöst hatte, wurde das Reaktionsgemisch auf ca. 50°C abgekühlt. Anschließend wurden unter Rühren 2,5 Mol Isophorondiisocyanat zugetropft, wobei die Reaktionstemperatur anstieg. Bei Rückflußtemperatur wurde das Reaktionsgemisch dann solange gerührt, bis der Isocyanatgruppengehalt des Gemisches praktisch konstant blieb. Die restlichen Isocyanatgruppen wurden durch Zusatz eines Amins, z.B. 2-Amino-2-methyl-1-propanol inaktiviert. Freie COOH-Gruppen wurden mit 2-Amino-2-methylpropanol neutralisiert. Anschließend wurde Wasser zugegeben und der größte Teil des Methylethylketons unter vermindertem Druck bei ca. 40°C entfernt. Man erhielt eine Dispersion des Polyurethans, die für die in dem nachfolgenden Beispiel 4 beschriebenen Versuche zum Vergleich verwendet wurde.

### Beispiel 3

### Bestimmung der Auswaschbarkeit der Polymere:

Die Auswaschbarkeit der gemäß Beispiel 1 erhaltenen Polymere mit Wasser wurde im Vergleich zu einem Polymer untersucht, bei dem die Isocyanatendgruppen nicht mit einer Aminosäure, sondern mit 2-Amino-2-methylpropanol umgesetzt wurden. Zu diesem Zweck wurde ein Film aus einer 5%igen wäßrigen und aus einer wäßrig-ethanolischen Dispersion (1:1 V/V) des Polymers auf einer Glasplatte erzeugt, in dem die Polymerdispersion auf die Glasplatte gegossen wurde. Man ließ 20 h bei Raumtemperatur trocknen. Die Auswaschbarkeit (Redispergierbarkeit) des Films, der aus Wasser oder Wasser/Ethanol (1:1 V/V) hergestellt wurde, wurde durch Rubbeln per Finger bestimmt. Die erhaltenen Ergebnisse sind in der nachfolgenden Tabelle angegeben.

**Tabelle:**

| Auswaschbarkeit der Polymeren | | | | |
|---|---|---|---|---|
| Versuch | K-Wert des Polyurethans | Aminosäure | Auswaschbarkeit | |
| | | | Film aus wäßriger Dispersion | Film aus H₂O/EtOH-Dispersion |
| 1* | 27 | -- | schlecht | schlecht |
| 2 | 27 | Asp | gut | gut |
| 3 | 27 | Glu | gut | gut |
| 4 | 27 | Tau | gut | gut |
| Asp = Asparaginsäure | | | | |
| Glu = Glutaminsäure | | | | |
| Tau = Taurin | | | | |

| | | | | |
|---|---|---|---|---|
| * Vergleich | | | | |

Es ist ersichtlich, daß die mit den erfindungsgemäßen Mitteln erhaltenen Filme überraschenderweise besser auswaschbar sind als der mit dem Mittel des Standes der Technik erhaltene Film.

### Beispiel 4

Haarspray-Formulierung mit einem VOC-Gehalt von 55 Gew.-%:

| Polyurethan gemäß Beispiel 1 | |
|---|---|
| (Feststoffgehalt) | 5,00 Gew.-% |
| Wasser | 40,00 Gew.-% |
| Ethanol | 25 Gew.-% |
| Dimethylether | 30 Gew.-% |
| Parfüm | q.s. |

Die Auswaschbarkeit des mit dieser Formulierung erhaltenen Films wurde im Vergleich zu einem Film bestimmt, der mit der gleichen Formulierung, die jedoch das Polyurethan gemäß Beispiel 2 enthielt, erhalten wurde. Die Auswaschbarkeit wurde an künstlichen Modellköpfen wie folgt bestimmt:

Das Haarspray wurde in einem Sprühvorgang von 10 sec (Auftragsmenge ca. 2,5 g) auf die Haare der Modellköpfe aufgetragen. Nach zweistündigem Trocknen im Klimaraum (Luftfeuchtigkeit 45 %; Temperatur 20°C) wurde die Festigungswirkung beurteilt. Dieser Vorgang wurde insgesamt 3 x wiederholt. Der besprühte Modellkopf wurde im Klimaraum über Nacht getrocknet. Danach wurde mit Texapon NSO nicht länger als 5 min shampooniert und gewaschen. Nach dem Trocknen wurde die Auswaschbarkeit von geschulten Fachleuten beurteilt. Es wurde gefunden, daß der mit der erfindungsgemäßen Formulierung erhaltene Film gut auswaschbar war, während sich der mit dem Polyurethan gemäß Beispiel 2 erhaltene Film nur schlecht auswaschen ließ.

## Patentansprüche

1. Wasserlösliche oder wasserdispergierbare Polyurethane aus
A) einem in Wasser löslichen oder dispergierbaren Polyurethanpräpolymer mit endständigen Isocyanatgruppen aus
a) mindestens einer Verbindung, die zwei oder mehrere aktive Wasserstoffatome pro Molekül enthält, ausgewählt unter Diolen, Diaminen, Aminoalkoholen, Polyetherdiolen,Polyesterdiolen, mit einem zahlenmittleren Molekulargewicht von jeweils bis zu 3000, oder deren Mischungen,
b) mindestens einer Verbindung, die zwei oder mehrer aktive Wasserstoffatome und mindestens eine ionogene bzw. ionische Gruppe pro Molekül aufweist, wobei es sich um Carboxylatgruppen oder um stickstoffhaltige Gruppen handelt, und
c) mindestens ein Diisocyanat, ausgewählt unter Isophorondiisocyanat, Methylendiphenyldiisocyanat und/oder Toluylendiisocyanat,
und
B) mindestens einem primären oder sekundären Amin, das wenigstens eine ionogene bzw. ionische Gruppe aufweist,
und die Salze davon.

2. Polyurethane nach Anspruch 1, wobei es sich bei der Komponente (a) um ein Polyesterdiol auf Basis einer aromatischen und einer aliphatischen Dicarbonsäure und eines aliphatischen Diols handelt.

3. Polyurethane nach Anspruch 2, wobei der Anteil der aromatischen Dicarbonsäure 10 bis 95 Mol-%, insbesondere 40 bis 90 Mol-% und besonders bevorzugt 50 bis 85 Mol-%, der gesamten Dicarbonsäuremenge ausmacht.

4. Polyurethane nach Anspruch 1, wobei das Verhältnis von NCO-Äquivalent der Verbindungen der Gruppe c) zu Äquivalent aktives Wasserstoffatom der Verbindungen der Gruppen a) und b) >1:1 bis 1,2:1 beträgt.

5. Polyurethane nach Anspruch 1, wobei es sich bei der Komponente b) um Dimethylolpropansäure handelt.

6. Polyurethane nach einem der Ansprüche 1 bis 5, wobei die Komponente a) mindestens 30 Gew.-%, insbesondere 40 bis 80 Gew.-%, bezogen auf das Gesamtgewicht der Komponenten a) und b), eines Polyesterdiols umfasst.

7. Polyurethane nach einem der Ansprüche 1 bis 6, wobei die Komponente a) bis zu 50 Gew.-%, bezogen auf das Gesamtgewicht der Komponenten a) und b), einer Silikonverbindung der Formel 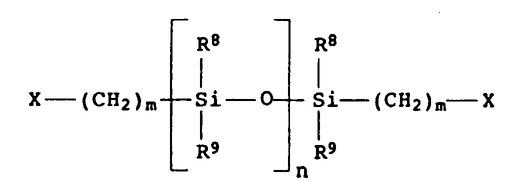 umfasst,
worin R⁸ und R⁹, die gleich oder verschieden sein können, für C₁-C₄-Alkyl, Benzyl oder Phenyl stehen,
die Reste X, die gleich oder verschieden sein können, für OH oder NH₂ stehen,
m für 2 bis 8 steht und
n für 3 bis 50 steht.

8. Polyurethane nach einem der vorhergehenden Ansprüche, wobei es sich bei der Komponente B) um eine Aminosulfonsäure der Formel
R¹⁰HN-Y-SO₃H
worin Y für o-, m oder p-Phenylen oder geradkettiges oder verzweigtes C₂-C₆-Alkylen steht, das gegebenenfalls durch 1, 2 oder 3 Hydroxygruppen substituiert ist, und
R¹⁰ für ein Wasserstoffatom, eine C₁-C₁₂-Alkylgruppe oder eine C₅-C₆-Cycloalkylgruppe steht, wobei die Alkyl- oder Cycloalkylgruppe gegebenenfalls durch eine, zwei oder 3 Hydroxygruppen, Carboxylgruppen oder Sulfonsäuregruppen substituiert sein kann,
oder um eine α-, β- oder γ-Aminocarbonsäure, insbesondere Asparaginsäure oder Glutaminsäure, handelt

9. Polyurethane nach Anspruch 8, wobei es sich bei der Aminosulfonsäure um Taurin, N-(1,1-Dimethyl-2-hydroxyethyl)-3-amino-2-hydroxypropansulfonsäure oder 4-Aminobenzolsulfonsäure handelt.

10. Verfahren zur Herstellung der Polyurethane nach einem der Ansprüche 1 bis 9, dadurch gekennzeichnet, dass man das Polyurethanpräpolymer mit dem primären oder sekundären Amin zur Reaktion bringt.

11. Verwendung der Polyurethane nach einem der Ansprüche 1 bis 9 als Hilfsmittel in der Kosmetik, insbesondere in der Haarkosmetik, und in der Pharmazie.

12. Kosmetisches oder pharmazeutisches Mittel, das wenigstens ein Polyurethan nach einem der Ansprüche 1 bis 9 umfasst.

## Claims

1. A water-soluble or water-dispersible polyurethane formed from
A) a water-soluble or -dispersible polyurethane prepolymer having terminal isocyanate groups formed from
a) at least one compound containing two or more active hydrogen atoms per molecule, selected from diols, diamines, amino alcohols, polyetherdiols and polyesterdiols, having a number-average molecular weight of in each case up to 3000, or mixtures thereof,
b) at least one compound containing two or more active hydrogen atoms and at least one ionogenic or ionic group per molecule, this group comprising carboxylate groups or nitrogen-containing groups and
c) at least one diisocyanate, selected from isophorone diisocyanate, methylenediphenyl diisocyanate and/or tolylene diisocyanate,
and
B) at least one primary or secondary amine having at least one ionogenic or ionic group,
or a salt thereof.

2. A polyurethane as claimed in claim 1, where component (a) is a polyesterdiol based on an aromatic and an aliphatic dicarboxylic acid and on an aliphatic diol.

3. A polyurethane as claimed in claim 2, where the fraction of the aromatic dicarboxylic acid makes up from 10 to 95 mol-%, in particular from 40 to 90 mol-% and, with particular preference, from 50 to 85 mol-% of the overall amount of dicarboxylic acid.

4. A polyurethane as claimed in claim 1, wherein the ratio of NCO equivalent of the compounds of group c) to equivalents of active hydrogen atom of the compounds of groups a) and b) is from > 1:1 to 1.2:1.

5. A polyurethane as claimed in claim 1, wherein component b) is dimethylolpropanoic acid.

6. A polyurethane as claimed in any one of claims 1 to 5, wherein component a) comprises at least 30% by weight, in particular from 40 to 80% by weight, based on the overall weight of components a) and b), of a polyesterdiol.

7. A polyurethane as claimed in any one of claims 1 to 6, wherein component a) comprises up to 50% by weight, based on the overall weight of components a) and b), of a silicone compound of the formula 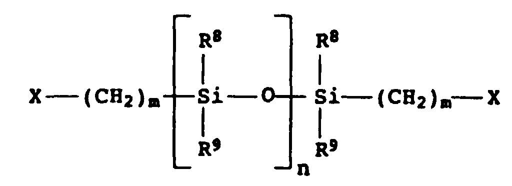
where R⁸ and R⁹ can be identical or different and are C₁-C₄-alkyl, benzyl or phenyl,
the radicals X can be identical or different and are OH or NH₂,
m is from 2 to 8, and
n is from 3 to 50.

8. A polyurethane as claimed in any one of the preceding claims, wherein component B) is an aminosulfonic acid of the formula
R¹⁰HN-Y-SO₃H
where Y is o-, m- or p-phenylene or straight-chain or branched C₂-C₆-alkylene which is unsubstituted or substituted by 1, 2 or 3 hydroxyl groups, and
R¹⁰ is hydrogen, C₁-C₁₂-alkyl or C₅-C₆-cycloalkyl, it being possible for the alkyl or cycloalkyl to be substituted, if desired, by 1, 2 or 3 hydroxyl, carboxyl or sulfo groups,
or is an α, β- or γ-aminocarboxylic acid, and in particular is aspartic acid or glutamic acid.

9. A polyurethane as claimed in claim 8, wherein the aminosulfonic acid is taurine, N-(1,1-dimethyl-2-hydroxyethyl)-3-amino-2-hydroxy-propanesulfonic acid or 4-aminobenzenesulfonic acid.

10. A process for preparing a polyurethane as claimed in any one of claims 1 to 9, which comprises reacting the polyurethane prepolymer with the primary or secondary amine.

11. The use of a polyurethane as claimed in any one of claims 1 to 9 as an auxiliary in cosmetology, especially in hair cosmetology, or in pharmacy.

12. A cosmetic or pharmaceutical composition which comprises at least one polyurethane as claimed in any one of claims 1 to 9.

## Revendications

1. Polyuréthanes solubles ou dispersables dans l'eau, constitués
A) d'un prépolymère de polyuréthane soluble ou dispersible dans l'eau, comportant des groupes isocyanate terminaux constitué
a) d'au moins un composé qui contient deux ou plusieurs atomes d'hydrogène actifs par molécule, choisi parmi les diols, les diamines, les amino-alcools, les polyétherdiols, les polyesterdiols, dont chacun a une masse moléculaire moyenne en nombre allant jusqu'à 3000, ou leurs mélanges,
b) d'au moins un composé comportant deux ou plusieurs atomes d'hydrogène actif et au moins un groupe ionogène ou ionique par molécule, auquel cas il s'agit de groupes carboxylate ou de groupes azotés, et
c) d'au moins un diisocyanate, choisi parmi le diisocyanate d'isophorone, le diisocyanate de méthylènediphényle et/ou le diisocyanate de toluylène,
et
B) d'au moins une amine primaire ou secondaire, qui comporte au moins un groupe ionogène ou ionique,
et leurs sels.

2. Polyuréthanes selon la revendication 1, dans lesquels, pour ce qui concerne le composant (a), il s'agit d'un polyesterdiol à base d'un acide dicarboxylique aromatique et d'un acide dicarboxylique aliphatique, et d'un diol aliphatique.

3. Polyuréthanes selon la revendication 2, dans lesquels la proportion de l'acide dicarboxylique aromatique est de 10 à 95 % en moles, en particulier de 40 à 90 % en moles et plus particulièrement de 50 à 85 % en moles, par rapport à la quantité totale d'acide dicarboxylique.

4. Polyuréthanes selon la revendication 1, dans lesquels le rapport entre les équivalents de NCO des composés du groupe c) et les équivalents d'atome d'hydrogène actif des composés des groupes a) et b) est >1:1 à 1,2:1.

5. Polyuréthanes selon la revendication 1, dans lesquels, pour ce qui est du composant b), il s'agit d'acide diméthylolpropanoique.

6. Polyuréthanes selon l'une des revendications 1 à 5, dans lesquels le composant a) contient au moins 30 % en poids, en particulier de 40 à 80 % en poids, par rapport au poids total des composants a) et b), d'un polyesterdiol.

7. Polyuréthanes selon l'une des revendications 1 à 6, dans lesquels le composant a) contient jusqu'à 50 % en poids, par rapport au poids total des composants a) et b), d'un composé du silicium de formule 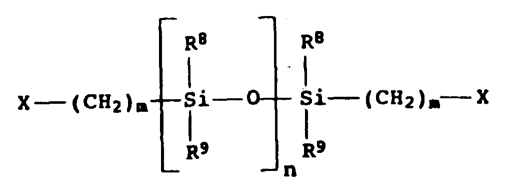 dans laquelle R⁸ et R⁹, qui peuvent identiques ou différents, sont des groupes alkyle en C₁-C₄, benzyle ou phényle,
les radicaux X, qui peuvent être identiques ou différents, représentent OH ou NH₂,
m vaut de 2 à 8, et
n vaut 3 à 50.

8. Polyuréthanes selon l'une des revendications précédentes, dans lesquels, pour ce qui est du composant B), il s'agit d'un acide aminosulfonique de formule
R¹⁰HN-Y-SO₃H
dans laquelle Y représente un radical o-, m- ou p-phénylène, ou alkylène en C₂-C₆ à chaîne droite ou ramifiée, qui est éventuellement substitué par 1, 2 ou 3 groupes hydroxy, et
R¹⁰ est un atome d'hydrogène, un groupe alkyle en C₁-C₁₂ ou un groupe cycloalkyle en C₅-C₆, où le groupe alkyle ou cycloalkyle peut être éventuellement substitué par 1, 2 ou 3 groupes hydroxy, groupes carboxyles ou groupes acide sulfonique,
ou encore d'un acide α, β- ou γ-aminocarboxylique, en particulier l'acide aspartique ou l'acide glutamique.

9. Polyuréthanes selon la revendication 8, dans lesquels, pour ce qui est de l'acide aminosulfonique, il s'agit de la taurine, de l'acide N-(1,1-diméthyl-2-hydroxyéthyl)-3-amino-2-hydroxypropanesulfonique ou de l'acide 4-aminobenzènesulfonique.

10. Procédé de préparation des polyuréthanes selon l'une des revendications 1 à 9, caractérisé en ce qu'on fait réagir le prépolymère de polyuréthane avec l'amine primaire ou secondaire.

11. Utilisation des polyuréthanes selon l'une des revendications 1 à 9 en tant qu'adjuvant en cosmétique, en particulier en cosmétique capillaire, et en pharmacie.

12. Produit cosmétique ou pharmaceutique, qui comprend au moins un polyuréthane selon l'une des revendications 1 à 9.
